# EUROPEAN PATENT APPLICATION

(11) **EP 2 267 126 A1**
(43) Date of publication of application: **29.12.2010**
(21) Application number: 09163894.0
(22) Date of filing: 26.06.2009
(51) Int. Cl.: C12N 15/09

(54) **Process for the stable gene interruption in clostridia**

(71) Applicant: Metabolic Explorer, 63360 Saint Beauzire (FR)
(72) Inventor: Soucaille, Philippe, 31450 DEYME (FR)
(74) Representative: Tetaz, Franck Claude Edouard

(57) **Abstract**

The present invention is related to a new method for interrupting multiple DNA sequences in *Clostridia,* even in genes recognized to be essential for the optimal growth of *Clostridii* by using a counter-selectable marker that would pinpoint the cells that have lost the plasmid and acquired a modified function that permits survival without the interrupted gene. This method is easy to perform and applicable at an industrial level. This method is useful to modify several genetic loci in *Clostridia* in a routine manner. This method is based on a replicative vector carrying at least two marker genes.

SEQUENCE LISTING

<110>  METABOLIC EXPLORER
       SOUCAILLE, PHILIPPE

<120>  PROCESS FOR A STABLE GENE INTERRUPTION IN CLOSTRIDIA

<130>  D27189

<160>  20

<170>  PatentIn version 3.5

<210>  1
<211>  53
<212>  DNA
<213>  Artificial Sequence

<220>
<223>  Oligonucleotide

<400>  1
aaaaaagctt ataattatcc ttacttgcct ctgaggtacg cccagatagg gtg          53

<210>  2
<211>  60
<212>  DNA
<213>  Artificial Sequence

<220>
<223>  Oligonucleotide

<400>  2
cagattgtac aaatgtggtg ataacagata agtctctgag attaacttac ctttctttgt    60

<210>  3
<211>  49
<212>  DNA
<213>  Artificial Sequence

<220>
<223>  Oligonucleotide

<400>  3
tgaacgcaag tttctaattt cggttgcaag tcgatagagg aaagtgtct               49

<210>  4
<211>  53
<212>  DNA
<213>  Artificial Sequence

<220>
<223>  Oligonucleotide

<400>  4
aaaaaagctt ataattatcc ttacccatct acacggtgcg cccagatagg gtg          53

<210>  5
<211>  60
<212>  DNA
<213>  Artificial Sequence

<220>
<223>  Oligonucleotide

<400>  5

cagattgtac aaatgtggtg ataacagata agtctacacg tctaacttac ctttctttgt        60

<210>  6
<211>  49
<212>  DNA
<213>  Artificial Sequence

<220>
<223>  Oligonucleotide

<400>  6
tgaacgcaag tttctaattt cggttatggg tcgatagagg aaagtgtct                   49

<210>  7
<211>  35
<212>  DNA
<213>  Artificial Sequence

<220>
<223>  Oligonucleotide

<400>  7
ggaacctcta taatatcctt cactccgtta attcc                                  35

<210>  8
<211>  31
<212>  DNA
<213>  Artificial Sequence

<220>
<223>  Oligonucleotide

<400>  8
ggatttgttg gttcttctac agtatttgcg c                                      31

<210>  9
<211>  53
<212>  DNA
<213>  Artificial Sequence

<220>
<223>  Oligonucleotide

<400>  9
aaaaaagctt ataattatcc ttagtactcg ctaaagtgcg cccagatagg gtg              53

<210>  10
<211>  60
<212>  DNA
<213>  Artificial Sequence

<220>
<223>  Oligonucleotide

<400>  10
cagattgtac aaatgtggtg ataacagata agtcgctaaa ggtaacttac ctttctttgt        60

<210>  11
<211>  49
<212>  DNA
<213>  Artificial Sequence

<220>
<223>  Oligonucleotide

<400>  11
tgaacgcaag tttctaattt cgattagtac tcgatagagg aaagtgtct                    49


<210>  12
<211>  53
<212>  DNA
<213>  Artificial Sequence

<220>
<223>  Oligonucleotide

<400>  12
aaaaaagctt ataattatcc ttatctaaca caagcgtgcg cccagatagg gtg               53


<210>  13
<211>  60
<212>  DNA
<213>  Artificial Sequence

<220>
<223>  Oligonucleotide

<400>  13
cagattgtac aaatgtggtg ataacagata agtcacaagc tttaacttac ctttctttgt        60


<210>  14
<211>  49
<212>  DNA
<213>  Artificial Sequence

<220>
<223>  Oligonucleotide

<400>  14
tgaacgcaag tttctaattt cggttttaga tcgatagagg aaagtgtct                    49


<210>  15
<211>  53
<212>  DNA
<213>  Artificial Sequence

<220>
<223>  Oligonucleotide

<400>  15
aaaaaagctt ataattatcc ttacaactca aattggtgcg cccagatagg gtg               53


<210>  16
<211>  60
<212>  DNA
<213>  Artificial Sequence

<220>
<223>  Oligonucleotide

<400>  16
cagattgtac aaatgtggtg ataacagata agtcaaattg gttaacttac ctttctttgt        60


<210>  17
<211>  49
<212>  DNA
<213>  Artificial Sequence

<220>
<223>  Oligonucleotide

<400>  17
tgaacgcaag tttctaattt cggttagttg tcgatagagg aaagtgtct                49


<210>  18
<211>  53
<212>  DNA
<213>  Artificial Sequence

<220>
<223>  Oligonucleotide

<400>  18
aaaaaagctt ataattatcc ttatgtatcc tggaagtgcg cccagatagg gtg           53


<210>  19
<211>  60
<212>  DNA
<213>  Artificial Sequence

<220>
<223>  Oligonucleotide

<400>  19
cagattgtac aaatgtggtg ataacagata agtcctggaa cataacttac ctttctttgt    60


<210>  20
<211>  49
<212>  DNA
<213>  Artificial Sequence

<220>
<223>  Oligonucleotide

<400>  20
tgaacgcaag tttctaattt cggttataca tcgatagagg aaagtgtct                49

## Description

### BACKGROUND OF THE INVENTION

*Clostridia* are low GC bacteria gram-positive bacteria with a strictly anaerobic life style. They are widely used in industry for their capacities to produce solvents, in particular butanol, ethanol and acetone, but also organic acids like acetic, butyric or lactic acid and vaccines. Furthermore, they are used for the production of diols like 1,3-propanediol.

To improve the industrial capabilities of *Clostridii,* the construction of genetically modified strains is an important part of the development in the field. However, *Clostridia* are not naturally transformable and currently available methods for their transformation are inefficient and do not permit the introduction of multiple mutations. This has hampered industrial developments in the field for a long time. Notably, *Clostridia* commonly produce extracellular DNAses and restriction enzymes that degrade foreign DNA before and after introduction into the cells for transformation. Classic methods based on the introduction of PCR fragments that work well in many microorganisms such as *Escherichia coli, Bacillus subtilis* or yeast, are not feasible in these organisms, since the extra- and intra-cellular half life of the DNA construct to be recombined is too short and recombination efficiency is generally low. In other organisms these difficulties have been circumvented by using vectors that replicate in the host thus increasing the likelihood of the recombination event. Nevertheless after the recombination event the vector that now carries the intact target DNA sequence has to be eliminated. This problem was solved in *Lactococcus lactis* (Biswas et al, J Bacteriol. 1993 Jun;175, :3628-35) by using temperature-sensitive replicons that can be eliminated at the non-permissive temperature. However, no vectors with temperature-sensitive replicons have been identified that could be used for *Clostridia.* Therefore construction of mutants in *Clostridia* has so far been very laborious and often unsuccessful.

Inactivations of genes in Clostridia were reported in the following articles and patent applications (see table 1).

**Table 1 :**

| Strain | Genotype | Reference |
|---|---|---|
| *Clostridium acetobutylicum* PJC4BK | *buk-, MLS^{R}* | Green *et al.,* 1996 |
| *Clostridium acetobutylicum* PJC4PTA | *pta-, MLS^{R}* | Green *et al.,* 1996 |
| *Clostridium acetobutylicum* PJC4AAD | *aad-, MLS^{R}* | Green and Bennett, 1998 |
| *Clostridium perfringens SM101 and F4969* | Δ*cpe, CatP* | Sarker et al., 1999 |
| *Clostridium perfringens Strain 13* | Δl*uxS* | Ohtani et al., 2002 |
| *Clostridium acetobutylicum* SK01 | Δ*spoA, MLS^{R}* | Harris *et al.,* 2002 |
| *Clostridium perfringens Type A* | Δ*spo0A* | Huang et al., 2004 |
| *Clostridium perfringensSM101* | *ccpA-, CatP* | Varga et al., 2004 |
| *Clostridium acetobutylicum* ATCC 824 buk- | *ΔSpollE, buk-, CatP* | WO 2006/007530 |

Up to date, gene inactivation was performed in *Clostridia* by transforming the cells with circular DNA that could not replicate in the target strains. Since the recombination frequency in this genus is not very high and the DNA degrading enzymes present in *Clostridia* rapidly break down the introduced DNA, obtaining *Clostridium* mutants has been very laborious.

Importantly, all the described recombinant strains (see above) are resistant to MLS or chloramphenicol and the corresponding marker genes can not be removed after the recombination event has occurred. This limits the number of possible recombination to the number of corresponding available resistance markers in these bacteria, which means a maximum of two.

Moreover, to use these recombinant strains for the industrial purposes, it is highly desirable to produce marker-less strains to prevent the release of antibiotic resistance genes into first fermentation media, and potentially the environment.

Finally, some of the strains that have been obtained by single recombination events using non-replicable plasmids have the disadvantage that they are not stable when cultured without any selection pressure, which requires constant feeding of antibiotics.

Recently, a method based on the insertion of group II introns into coding sequences allowed for the fast production of mutants in four different species (Heap et al., 2007). Although the method chosen allowed for rapid screening of the insertion of the introns, it leaves behind an antibiotic resistance marker which prevents the reuse of the system for multiple gene inactivation in the same strain. Use of the intron has also been reported without antibiotic marker left in the genome of the mutant strain (Shao et al., 2007). However, multiple deletions require several runs of *Clostridium* transformation and selection for intron insertion, followed by plasmid loss. Inactivation of genes in Clostridia by intron insertion are listed in table 2.

**Table 2:**

| Strain | Genotype | Reference |
|---|---|---|
| *C. acetobutylicum* spo0A | *spo0A- MLS^{R}* | Heap et al., 2007 |
| *C. acetobutylicum* pyrF | *pyrF- MLS^{R}* | Heap et al., 2007 |
| *C. acetobutylicum* CAC0081 | CAC0081- *MLS^{R}* | Heap et al., 2007 |
| *C. acetobutylicum* CAC0080 | CAC0080- *MLS^{R}* | Heap et al., 2007 |
| *C. acetobutylicum* CAC0078 | CAC0078- *MLS^{R}* | Heap et al., 2007 |
| *C. acetobutylicum* CAC2208 | CAC2208- *MLS^{R}* | Heap et al., 2007 |
| *C. difficile* spo0A | *spo0A- MLS^{R}* | Heap et al., 2007 |
| *C. difficile* pyrF | *pyrF- MLS^{R}* | Heap et al., 2007 |
| *C. difficile* CD0153 | CD0153- *MLS^{R}* | Heap et al., 2007 |
| *C. difficile* CD0552 | CD0552- *MLS^{R}* | Heap et al., 2007 |
| *C. difficile* CD3563 | CD3563- *MLS^{R}* | Heap et al., 2007 |
| *C. sporogenes* spo0A | *spo0A- MLS^{R}* | Heap et al., 2007 |
| *C. sporogenes* pyrF | *pyrF- MLS^{R}* | Heap et al., 2007 |
| *C. sporogenes* CBO2641 homolog | CBO2641-MLS^{R} | Heap et al., 2007 |
| *C. sporogenes* CBO3558 homolog | CBO3558- *MLS^{R}* | Heap et al., 2007 |
| *C. botulinum* CBO0332Ad | CBO0332Ad- *MLS^{R}* | Heap et al., 2007 |
| *C. botulinum* CBO0339 | CBO0339- *MLS^{R}* | Heap et al., 2007 |
| *C. botulinum* CBO0336 | CBO0336- *MLS^{R}* | Heap et al., 2007 |
| *C. botulinum* CBO0340 | CBO0340- *MLS^{R}* | Heap et al., 2007 |
| *C. acetobutylicum* | *buk-* | Shao et al., 2007 |
| *C. acetobutylicum* | *solR-* | Shao et al., 2007 |

Intron insertion in a coding sequence can occur in two different ways.

In the first option, the insertion can be targeted in the coding DNA strand, which is later transcribed as fully operational type II intron, namely the forward transcription orientation. This RNA sequence is then recognized by the reverse transcriptase carried by the vector used for the initial insertion, which can extract the intron and close the messenger RNA, allowing the expression of the encoded protein.

According to the second option, the insertion can be targeted to the non-coding DNA strand, namely the reverse transcription orientation. In this case, the intron sequence is transcribed in the opposite direction and the sequence can not be recognized, leading to stable interruption even in the presence of the reverse transcriptase carried by the vector. In the case of the insertion of the intron in a sequence coding for a protein essential for the growth of the organism, the reverse transcription orientation will be lethal to the desired mutant strain. The forward transcription orientation insertion will then be the only possible intron insertion event achievable, but the final desired phenotype will not appear. A strain carrying the vector for forward transcription oriented vector will be able to survive, and avoid the loss of the activity carried by the targeted gene, as long as the plasmid remains in the cells. However, it is conceivable, for example, that because of the delayed transcription of the targeted gene, some cells mutate in order to bypass the insertion, in which case the plasmid can be lost in the absence of the antibiotic selective pressure. However these mutation events will be rare because the pressure for the appearance of bypass mutations is weak. Therefore, the number of cells that will loose the plasmid is low and selection of such cells will require long and tedious analyses of a large number of clones without a very efficient screening method.

Importantly, the *C. acetobutylicum buk-* strain obtained by Shao et al., was constructed using a forward transcription orientation and no indication of the loss of the plasmid was given in the publication. Later, oral presentation by Sheng Yang at the Clostridium 10 Workshop held in Wageningen (The Netherlands) from September 28 to October 1, 2008, indicated that indeed the vector carrying the butyrate kinase targeted intron could not be lost (http://www.clostridium10.org/everyone). The authors had to create a new plasmid (pSY7-*buk*) in which the MLSr resistance marker was replaced by the Thiamphenicol resistance cassette to combine two mutations. The strain, containing the double interruption using the vectors pSY6-solR and the pSY7-*buk,* never lost the pSY7-*buk* vector and potentially still produced butyrate.

As a result, there is a strong need in the state of the art for a method to genetically modify *Clostridii* with high efficiency, with an easy step of strains selection that would allow concomitant DNA sequence interruption in the same strain. The selection of mutant strains with the desired phenotype can be made by positive selection of the rare events that would allow survival of an otherwise undetectable phenotype, leading to genetically modified *Clostridia* that are genetically stable and marker-less.

The present invention is related to a new method for interrupting multiple DNA sequences in *Clostridia,* even in genes recognized to be essential for the optimal growth of *Clostridii* by using a counter-selectable marker that would pinpoint the cells that have 1) been submitted to the interruption of the gene, and 2) lost the plasmid carrying the marker and acquired a modified function that permits survival without the interrupted gene. This method is easy to perform and applicable at an industrial level. This method is useful to modify several genetic loci in *Clostridia* in a routine manner.

This method is based on a replicative vector useful for the transformation of *Clostridia* with high efficiency. An unlimited number of mutations can be introduced into the genome with this new method, avoiding resistance cassettes insertion in the genome, positively selecting for the loss of the replicative vector, allowing the reuse of the system in successive rounds of DNA sequence interruption if necessary.

Efficient introduction of multiple mutations into *Clostridia* should enable industry to improve existing industrial strains and to develop new processes.

### DESCRIPTION OF THE INVENTION

The invention provides a process for the stable interruption of a target gene in a strain of the type Clostridia comprising:
i. Transforming said strain with a replicative vector containing:
   - An origin of replication functional in Clostridia
   - A first marker gene
   - A second marker gene
   - One or more group II intron sequences
   - A reverse transcriptase encoding gene.
ii. Selecting strains having integrated said replicative vector that express the second marker gene.
iii. Selecting strains having an interrupted gene following the integration of the intron sequence into said gene.
iv. Optionally, culturing the selected strains to obtain their evolution toward a state wherein they are able to survive with the interrupted gene.
v. Selecting strains having eliminated said replicative vector that do not express the first and the second marker genes.

All molecular biology techniques used for realizing the invention are fully described in « Molecular cloning: a laboratory manual », 2nd edition, Cold Spring Harbor Laboratory Press, 1989, by Sambrook, Fritsch and Maniatis.

Used in the context of the present invention, the term « interruption » of a target DNA sequence means that a sequence is introduced at the locus of the target DNA sequence. The expression "stable interruption" means that the interruption of the gene will stay into the strain for at least 50 generations and even more.

According to the invention a DNA sequence is defined as a gene, or an intergenic sequence. Both sequences can comprise promoter or regulatory sequences.

The expression « target gene » means any gene, intergenic region, promoter or regulatory sequence of interest chosen by the man skilled in the art. It means in particular genes coding for proteins of interest, for example enzymes involved in the cellular metabolism.

Due to the insertion of type II Introns, the expression of the target gene is usually perturbed, partially or completely suppressed.

The term « transformation » refers to the incorporation of exogenous nucleic acid by a cell.

The term « replicative vector » refers to a vector, i.e. an extra-chromosomal element, carrying genes or cassettes, and that is usually in the form of a circular double-stranded DNA molecule, but may be a single strand DNA molecule, too. Both terms vector and plasmid are used to designate the same thing. The used vector is a replicative vector. It comprises at least one origin of replication, permitting its replication in *Clostridia,* and preferentially several origins of replication, allowing it to be functional in different species.

In particular a preferred vector may comprise two replicative origins:
- Ori, functional in *E. coli,*
- RepL from pIM13 issued of *B. subtilis,* functional in *Clostridia* (Mermelstein et al, Biotechnology, 1992, 10, 190-5).

The used vector also contains a reverse transcriptase encoding gene. The presence of a reverse transcriptase is essential for the insertion of the intron sequence into the targeted gene. Indeed, the reverse transcriptase associate itself with the RNA transcribed from the intron sequence and then insert this RNA molecule into the targeted gene.

The term 'group II intron sequences' designates sequences of bacterial mobile group II intron, originating from the Lactococcus lactis L1.LtrB intron.

The term 'reverse transcriptase' designates an enzyme that catalyzes the transcription of RNA (ribonucleic acid) into DNA (deoxyribonucleic acid). This catalyzed transcription is the reverse process of normal cellular transcription of DNA into RNA.

The term 'selection' according to the invention denotes a process wherein the only strains of microorganisms that are retained in the culture medium are those presenting a better fitness under the selection pressure conditions. Typically, the fittest strains are outgrowing their competitors and are then selected. A simple way to select a specific evolved strain of microorganism in a population consists in growing the population in continuous culture in which slow-growing strains are eventually eluted from the culture. This is not an exclusive example for selection, and other methods of selection known by the expert in the field may be applied.

The term « marker gene » refers to a sequence coding for a marker protein under the control of regulatory elements functional in *Clostridia.* Such proteins are well known in the art. For example the man skilled in the art may use an antibiotic resistance gene, a fluorescent or colored marker, or a marker of auxotrophy. Examples of useful marker genes will be given below.

After the insertion event has occurred, the vector has to be eliminated. The elimination of a replicative vector generally happens with successive cultures of clones, followed by negative or positive selection of clones having eliminated this vector. Once the vector is cured, strains do not express the marker genes anymore and can be selected on this characteristic.

In a particular embodiment of the invention, the DNA molecule contains one or more group II intron sequences that are operably linked to the same promoter for expression in Clostridia. This characteristic allows the interruption of multiple genes at once, eliminating the necessity to cure the newly obtained strain from the plasmid before starting the interruption of a new gene.

In said DNA molecule, at least one group II intron sequence may be oriented for forward transcription.

In another embodiment of the invention, at least one group II intron sequence is oriented for reverse transcription.

When the type II intron has been integrated with a forward transcription orientation, it is then transcribed as RNA. This RNA sequence is then recognized by the reverse transcriptase carried by the replicative vector, which can extract the intron and close the messenger RNA, allowing the expression of the protein encoded by the interrupted gene. Therefore, the gene will be stably interrupted only after the curing of the vector. The curing of the vector might be delayed up to the point wherein the strain has evolved to be able to survive with the interrupted gene. In this case, an optional step of culturing the selected strains to obtain their evolution is performed during the process.

When the type II intron has been integrated with a reverse transcription orientation, the intron sequence is transcribed in the opposite direction and the sequence can not be recognized by the reverse transcriptase, leading to immediate stable interruption of the gene at the DNA but also at the RNA levels.

If the interruption targets a gene coding for a protein essential for the strain survival, the reverse transcription orientation will be lethal for the mutant strain. In this case, the forward transcription orientation insertion is interesting since the protein might be expressed for a while, until the strain evolve, before the curing of the vector.

The evolution process according to a particular embodiment of the invention is a process for the preparation of evolved strains presenting the ability to survive without the interrupted gene, and comprises the following steps:
a) Interruption of a gene, as described,
b) Growth of the strains obtained above on an appropriate medium in order to cause them to evolve, wherein the said strains are grown under aerobic, micro-aerobic or anaerobic conditions,
c) Selection of the "evolved strains" able to survive under these specific conditions.

The general evolution process and appropriate medium have been extensively described in the patent applications WO 2004/076659 filed on 17/02/2004, and WO 2005/073364 filed on 12/01/2005, by the same applicants.

In a particular embodiment of the invention, the first marker gene is an antibiotic resistance gene.

Among the useful antibiotic resistance genes, the man skilled in the art would know which is the most appropriate. For example the following genes may be used: CatP gene, giving the resistance to chloramphenicol and thiamphenicol, or MLSR gene, giving resistance to erythromycin.

In a preferred embodiment of the invention, the second marker gene is a counter-selectable marker gene.

A counter-selectable marker is a gene whose presence is lethal to the host organism under certain circumstances such as the presence of its cognate substrate. Counter-selectable markers can be used as a positive selection for the loss of the plasmid.

Preferentially the counter-selectable marker gene is a gene that restores the activity of an absent or deleted non-essential endogenous gene.

The most-used counter-selectable markers are the genes that confer sucrose, streptomycin, or fusaric acid sensitivity. They have been used to construct mutants or vaccine strains in several bacterial strains. For details see the review by Reyrat et at., 1998, Infection and Immunity. Counter-selectable markers that can also be used in *Clostridia* include gene giving sensitivity to 5-fluoro-uracile (5-FU), 5-fluoro-cytosine (5-FC), gamma-glutamyl hydrazide (GBS) or 8-aza-2,6-diaminopurine (8ADP).

In a preferred embodiment, the counter-selectable marker is the *upp* gene, which encodes uracil phosphoribosyl-transferase that promotes transformation of 5-fluoro-uracile (5-FU) to a toxic product. Cells having Upp activity transform the 5-FU to a toxic product and can not grow on a 5-FU medium.

The use of this counter-selectable marker is particularly useful when the transformed *Clostridia* are deleted for the upp gene (Δ*upp*), and consequently are able to grow on a medium comprising 5-FU before the transformation and after the elimination of the vector. Strains having eliminated the vector can be positively selected.

Suppressor mutants that may arise in the *upp* gene in the presence of 5-FU, can sometimes lead to false assumptions with respect to the loss of the plasmid. In a preferred embodiment of the invention, the first marker gene, preferentially an antibiotic resistance gene permits a second selection of strains sensitive to the antibiotic. This negative selection may be used in the complementation of the positive selection based on the *upp* gene.

In another embodiment, the counter-selectable marker is the *E. coli codA* gene or an homologous gene having cytosine deaminase activity that promotes transformation of 5-fluoro-cytosine (5-FC) to 5-FU.

The use of codA gene as counter-selectable marker is particularly useful when the transformed *Clostridia* have an intact *upp* gene, and consequently are able to grow on a medium comprising 5-FC before the transformation and after the elimination of the vector. Strains having eliminated the vector can be positively selected.

Advantageously, the method according to the invention, for introducing group II intron in forward or reverse transcription orientation into a site of the chromosome of Clostridia strains, comprises the following steps:
a) Providing the strains having integrated the replicative vector as described above
b) Culturing the transformed strains under conditions which allow for the selection of said strains containing the replicative vector
c) Analyzing selected strains by PCR to confirm the gene interruption by the type II intron
d) Culturing the selected strains under conditions which allow for the loss of the replicative vector.

Advantageously, the step of culturing the bacterial cells allows defining the essentiality of a gene for optimal growth and survival of the bacterial strain.

In a preferred embodiment of the invention, the method described above further comprises the analysis of the fermentation broth resulting of the culture of strains, to establish the desired change in products ratio.

In an advantageous embodiment of the invention, the *Clostridium strains* to be transformed are deleted for the genes encoding restriction endonucleases. These strains present the advantage that they are readily transformable without any prior *in vivo* plasmid methylation.

In another advantageous embodiment of the invention, the *Clostridium strains* to be transformed are deleted for the genes encoding the extra-cellular DNAses.

In another embodiment of the invention, the *Clostridia* to be transformed are deleted for the *upp* gene.

In another advantageous embodiment of the invention the *Clostridium* strains are chosen among *Clostridium acetobutylicum, Clostridium bejeirinckii, Clostridium saccharoperbutylacetonicum, Clostridium butylicum, Clostridium butyricum, Clostridium perfringens, Clostridium tetani, Clostridium sporogenes, Clostridium thermocellum, Clostridium saccharolyticum (now Thermoanaerobacter saccharolyticum), Clostridium thermosulfurogenes (now Thermoanaerobacter thermosulfurigenes), Clostridium thermohydrosulfuricum (now Thermoanaerobacter ethanolicus).*

The preferred *Clostridium* strain is *Clostridium acetobutylicum.*

In a preferred embodiment of the invention, the *Clostridium acetobutylicum* strain to be transformed is a Δ*cac*15 strain, deleted for the gene encoding for the restriction endonuclease Cac 8241. This strain presents the advantage that it is readily transformable without any prior *in vivo* plasmid methylation.

In another preferred embodiment of the invention, the *Clostridium acetobutylicum* strain to be transformed is a strain whose *upp* gene has been deleted, termed Δ*upp.* As a consequence the transformation of the strain with a vector carrying the *upp* gene allows the selection of strains sensible to 5-FU medium, and then the positive selection of strains having lost the plasmid that are not sensible to 5-FU medium anymore.

The *Clostridium acetobutylicum* strain to be transformed can also be Δ*cac1*5 Δ*upp.*

The process is advantageously used for successive interruption of two or more target genes in the same *Clostridium* strain.

The present invention also concerns a recombinant *Clostridium* strain susceptible to be obtained by the process according to the invention. Advantageously, the process according to the invention may be used to obtain recombinant *Clostridium* strains wherein the gene *cac*5 was deleted strain Δ*cac*15), wherein the gene *upp* was deleted (strain Δ*upp*) and wherein both genes *cac*15 and *upp* were deleted (strain Δ*cac*15 Δ*upp*).

The present invention also concerns the replicative vector for transforming the strain, such as described above.

### DESCRIPTION OF DRAWINGS

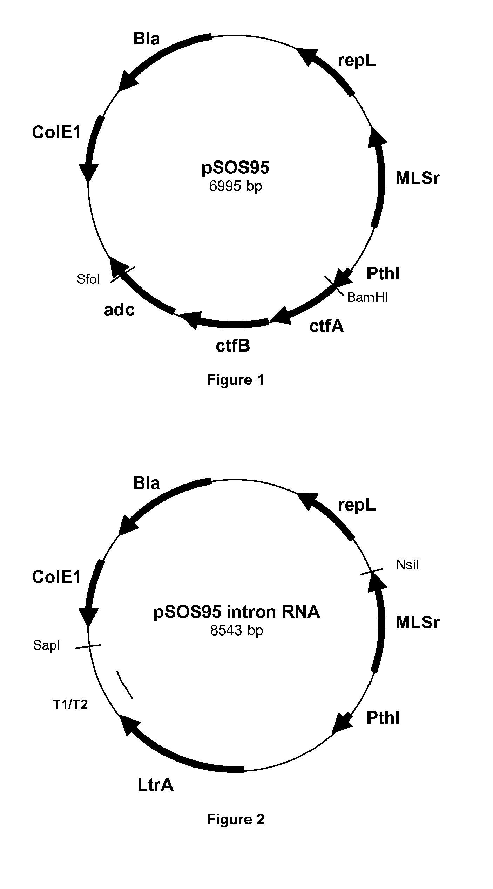

**Figure 1****:** Map of the pSOS95 plasmid.
**Figure 2****:** Map of the pSOS95 intron RNA.
**Figure 3****:** Map of the pCons::upp 46.
**Figure 4****:** Interruption of the *Idh* gene using the antisens intron. Line 1, *Idh* gene interrupted in anti-sens orientation by the Intron (416 pb); line 2, *Idh* amplification in the wild type strain (No amplification); line 3, molecular marker. The sizes of the molecular marker bands are indicated.

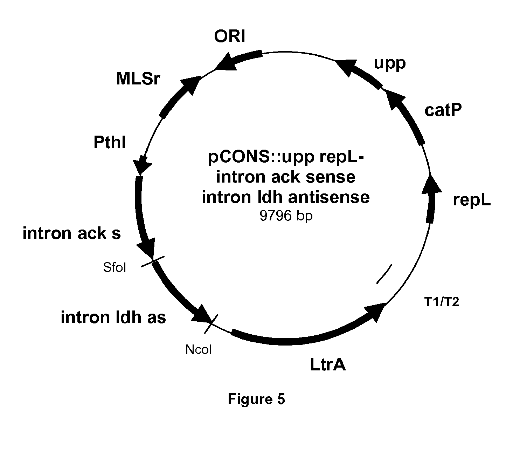

**Figure 5****:** Map of the pCONS::upp repL- intron ack sense - intron *Idh* antisense.

### EXAMPLES

### EXAMPLE 1

### Contruction of the pCONS::upp-intron vector

This plasmid contains a pIM13 origin of replication fuctional in Clostridia, a *catP* gene conferring resistance to thiamphénicol, the *upp* gene and LtrA ORF required for functional expression of the group II intron RNP. In order to construct the pCONS-intron vector, we sub-cloned the sequence of the LtrA ORF into the pCONS::upp vector. The LtrA ORF region was obtained from restriction digestion of pACD4 vector (Sigma TargeTron) with *XbaI* and *PshAI.* The pSOS95 vector was digested with *BamHI* and *SfoI* blunt ended to remove the acetone formation genes while leaving the thiolase promoter region. The LtrA ORF digest product and the linearized pSOS95 vector were ligated to create the pSOS-intron vector.

The pSOS-intron vector was digested by *NsiI* and *SapI* to remove the thiolase promoter and LtrA ORF. The pCONS::upp was digested with *SapI* and blunt ended. These fragments were ligated together to generate the pCONS::upp-intron vector.

### EXAMPLE 2

### Contruction of the pCONS::upp-intron ldh sense and pCONS::upp-intron ldh antisense vectors

To inactivate the *Idh* gene, a strain with the insertion of sense or antisense intron II in the *Idh* gene was constructed as follows. First, a computer algorithm was used to identify target sites in the *Idh* gene. Second, the computer algorithm outputs primer sequences (Table 2) which are used to mutate (re-target) the Idh sense intron by PCR with the primers LDH 1, LDH 2, LDH 3 and the EBS universal primer or the Idh antisense intron by PCR with the primers LDH 4, LDH 5, LDH 6 and the EBS universal primer. Next, the mutated 350 pb PCR fragment Idh sense or antisense intron and the pCONS::upp-intron vector were digested by *BsrGI* and *HindIII* and then ligated to yield the pCONS::upp-intron Idh sense or the pCONS::upp-intron Idh antisense.

**Table 3: primers sequences**

| Name | | Primer sequences |
|---|---|---|
| LDH 1(IBS) | SEQ ID N°1 | aaaaaagcttataattatccttacttgcctctgaggtacgcccagatagggtg |
| LDH 2 (EBS1d) | SEQ ID N°2 | cagattgtacaaatgtggtgataacagataagtctctgagattaacttacctttctttgt |
| LDH 3 (EBS2) | SEQ ID N°3 | tgaacgcaagtttctaatttcggttgcaagtcgatagaggaaagtgtct |
| LDH 4(IBS) | SEQ ID N°4 | aaaaaagcttataattatccttacccatctacacggtgcgcccagatagggtg |
| LDH 5 (EBS1d) | SEQ ID N°5 | cagattgtacaaatgtggtgataacagataagtctacacgtctaacttacctttctttgt |
| LDH 6 (EBS2) | SEQ ID N°6 | tgaacgcaagtttctaatttcggttatgggtcgatagaggaaagtgtct |
| LDH 7 | SEQ ID N°7 | ggaacctctataatatccttcactccgttaattcc |
| LDH 8 | SEQ ID N°8 | ggatttgttggttcttctacagtatttgcgc |

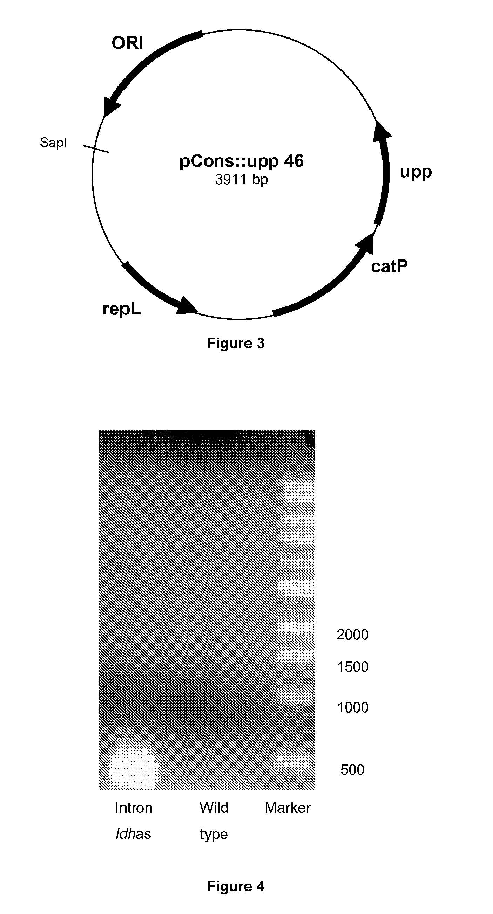

The pCONS::upp-intron Idh antisense plasmid was used to transform by electroporation a *C. acetobutylicum* strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The genotype of clones resistant to thiamphenicol was checked by PCR analysis with primers LDH 7 and LDH 8 (Table 3) located outside of the *Idh* gene (Figure 4). The *C. acetobutylicum Idh::*intron II strain which has lost pCONS::upp-intron Idh antisense was isolated on RCA with 5-FU at 400 µM.

The pCONS::upp-intron Idh sense plasmid was used to transform by electroporation a *C. acetobutylicum* strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The genotype of clones resistant to thiamphenicol was checked by PCR analysis with primers LDH 7 and LDH 8 located outside of the *Idh* gene. The *C. acetobutylicum Idh::*intron II strain which has lost pCONS::upp-intron Idh antisense was isolated on RCA with 5-FU at 400 µM.

### EXAMPLE 3

### Double intron integration

The pCONS::upp-intron ack sense- intron Idh antisense vector was constructed using specific primers described in table 3 and 4.

**Table 4: primers sequences**

| Name | | Primer sequences |
|---|---|---|
| ACK 1(IBS) | SEQ ID N°9 | aaaaaagcttataattatccttagtactcgctaaagtgcgcccagatagggtg |
| ACK 2 (EBS1d) | SEQ ID N°10 | cagattgtacaaatgtggtgataacagataagtcgctaaaggtaacttacctttctttgt |
| ACK 3 (EBS2) | SEQ ID N°11 | tgaacgcaagtttctaatttcgattagtactcgatagaggaaagtgtct |
| ACK 4(IBS) | SEQ ID N°12 | aaaaaagcttataattatccttatctaacacaagcgtgcgcccagatagggtg |
| ACK 5 (EBS1d) | SEQ ID N°13 | cagattgtacaaatgtggtgataacagataagtcacaagctttaacttacctttctttgt |
| ACK 6 (EBS2) | SEQ ID N°14 | tgaacgcaagtttctaatttcggttttagatcgatagaggaaagtgtct |

The pCONS::upp-intron ack sense- intron Idh antisense plasmid was then used to transform by electroporation a *C. acetobutylicum* strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The genotype of clones resistant to thiamphenicol was checked by PCR analysis located outside of the *Idh* and ack genes and show integration in both genes.

### EXAMPLE 4

### Butyrate kinase inactivation

To inactivate the *buk* gene, a strain with the insertion of sense or antisense intron II in the *buk* gene was constructed as follows. First, a computer algorithm was used to identify target sites in the *buk* gene. Second, the computer algorithm outputs primer sequences (Table 5) which are used to mutate (re-target) the buk sense intron by PCR with the primers BUK 1, BUK 2, BUK 3 and the EBS universal primer or the buk antisense intron by PCR with the primers BUK 4, BUK 5, BUK 6 and the EBS universal primer. Next, the mutated 350 pb PCR fragment buk sense or antisense intron and the pCONS::upp-intron vector were digested by *BsrGI* and *HindIII* and then ligated to yield the pCONS::upp-intron buk sense or the pCONS::upp-intron buk antisense.

**Table 5: primers sequences**

| Name | | Primer sequences |
|---|---|---|
| BUK 1(IBS) | SEQ ID N°15 | aaaaaagcttataattatccttacaactcaaattggtgcgcccagatagggtg |
| BUK 2 (EBS1d) | SEQ ID N°16 | cagattgtacaaatgtggtgataacagataagtcaaattggttaacttacctttctttgt |
| BUK 3 (EBS2) | SEQ ID N°17 | tgaacgcaagtttctaatttcggttagttgtcgatagaggaaagtgtct |
| BUK 4(IBS) | SEQ ID N°18 | aaaaaagcttataattatccttatgtatcctggaagtgcgcccagatagggtg |
| BUK 5 (EBS1d) | SEQ ID N°19 | cagattgtacaaatgtggtgataacagataagtcctggaacataacttacctttctttgt |
| BUK 6 (EBS2) | SEQ ID N°20 | tgaacgcaagtttctaatttcggttatacatcgatagaggaaagtgtct |

The pCONS::upp-intron buk sense or the pCONS::upp-intron buk antisense plasmid were used to transform by electroporation a *C. acetobutylicum* strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The genotype of clones resistant to thiamphenicol was checked by PCR analysis located outside of the *buk* gene. Interruption of the *buk* gene using the intron in reverse transcriptional orientation could not be obtained. On the contrary, interruption of the *buk* gene using the intron in forward transcriptional orientation could be obtained, suggesting the importance of the butyrate kinase for the fitness of the strain.

One colony of the *C. acetobutylicum buk::*intron II pCONS::upp-intron buk sense strain was then cultured in chemostat on synthetic medium without antibiotic. Serial dilution of the culture was regularly plated on 2YTG plates containing 5-Fluorouracyl (5-FU). Several clones were obtained which were resistant to 5-FU. When transferred on 2YTG plates plus or minus thiamphenicol, a large number of these clones did show thiamphenicol resistance, suggesting they still contained the pCONS::upp-intron buk sense with a mutation in the *upp* gene, conferring resistance to 5-FU. However, some clones could be shown to be thiamphenicol sensitive. We could confirm that these last clones did not contain the pCONS::upp-intron buk sense plasmid but still had the insertion of the intron II in the *buk* gene. We could therefore easily select for a complementary mutation allowing for the inactivation of the butyrate kinase in this strain. This mutation was identified as a point mutation in the phospho-transbutyrilase gene *ptb* which converts butyryl-CoA into butyryl-phosphate, the substrate of the butyrate kinase.

### REFERENCES

Green EM, Bennett GN. 1996. Inactivation of an aldehyde/alcohol dehydrogenase gene from Clostridium acetobutylicum ATCC 824. Appl Biochem Biotechnol. 57-58: 213-21.
Green EM, Bennett GN. 1998. Genetic manipulation of acid and solvent formation in Clostridium acetobutylicum ATCC 824. Biotechnol Bioeng. 58: 215-21.
Harris LM, Welker NE, Papoutsakis ET. 2002. Northern, morphological, and fermentation analysis of spo0A inactivation and overexpression in Clostridium acetobutylicum ATCC 824. J Bacteriol. 184: 3586-97.
Heap JT, Pennington OJ, Cartman ST, Carter GP, Minton NP. 2007. The ClosTron: a universal gene knock-out system for the genus Clostridium. J Microbiol Methods. 70: 452-64.
Huang IH, Waters M, Grau RR, Sarker MR. 2004. Disruption of the gene (spo0A) encoding sporulation transcription factor blocks endospore formation and enterotoxin production in enterotoxigenic Clostridium perfringens type A. FEMS Microbiol Lett. 233: 233-40.
Ohtani K, Hayashi H, Shimizu T. 2002. The luxS gene is involved in cell-cell signalling for toxin production in Clostridium perfringens. Mol Microbiol. 44: 171-9.
Sarker MR, Carman RJ, McClane BA. 1999. Inactivation of the gene (cpe) encoding Clostridium perfringens enterotoxin eliminates the ability of two cpe-positive C. perfringens type A human gastrointestinal disease isolates to affect rabbit ileal loops. Mol Microbiol. 33: 946-58.
Shao L, Hu S, Yang Y, Gu Y, Chen J, Yang Y, Jiang W, Yang S. 2007. Targeted gene disruption by use of a group II intron (targetron) vector in Clostridium acetobutylicum. Cell Res. 17: 963-5.
Varga J, Stirewalt VL, Melville SB. 2004. The CcpA protein is necessary for efficient sporulation and enterotoxin gene (cpe) regulation in Clostridium perfringens. J Bacteriol. 186: 5221-9.

## Claims

1. A process for the stable interruption of a target gene in a strain of the type Clostridia comprising:
i. Transforming said strain with a replicative vector containing:
- An origin of replication functional in Clostridia
- A first marker gene
- A second marker gene
- One or more group II intron sequences
- A reverse transcriptase encoding gene.
ii. Selecting strains having integrated said replicative vector that express the second marker gene.
iii. Selecting strains having an interrupted gene following the integration of the intron sequence into said gene.
iv. Optionally, culturing the selected strains to obtain their evolution toward a state wherein they are able to survive with the interrupted gene.
v. Selecting strains having eliminated said replicative vector that do not express the first and the second marker genes.

2. The process according to claim 1 wherein the replicative vector contains one or more group II intron sequence operably linked to the same promoter for expression in Clostridial cells.

3. The process according to claims 1 or 2 wherein the replicative vector contains at least one group II intron sequence that is oriented for forward transcription.

4. The process according to claim 3 wherein the optional step (iv) of culturing the selected strains to obtain their evolution is performed.

5. The process according to claim 1 or 2 wherein the replicative vector contains at least one group II intron sequence oriented for reverse transcription.

6. The process according to claim 1 to 5 wherein the first selection marker gene is an antibiotic resistance marker.

7. The process according to claim 1 to 6 wherein the second marker gene is a counter-selectable marker gene.

8. The process according to claim 7 wherein the counter-selectable marker is a gene that restores the activity of a non essential, absent or deleted gene.

9. The process according to claim 8, wherein the counter selectable marker is the *upp* or the codA gene.

10. The process according to anyone of claims 1 to 9, wherein the Clostridia to be transformed are deleted for the *upp* gene.

11. The process according to anyone of claims 1 to 10, wherein the Clostridium strains are chosen among *Clostridium acetobutylicum, Clostridium bejeirinckii, Clostridium saccharoperbutylacetonicum, Clostridium butylicum, Clostridium butyricum, Clostridium perfringens, Clostridium tetani, Clostridium sporogenes, Clostridium thermocellum, Clostridium saccharolyticum* (now *Thermoanaerobacter saccharolyticum*), *Clostridium thermosulfurogenes* (now *Thermoanaerobacter thermosulfurigenes*), *Clostridium thermohydrosulfuricum* (now *Thermoanaerobacter ethanolicus*).

12. The process according to claim 11 wherein the *Clostridium* strain is *Clostridium acetobutylicum* and the strain is Δ*upp.*

13. Recombinant *Clostridium* strains susceptible to be obtained by the process according to claims 1 to 12.

14. A replicative vector such as defined in anyone of claims 1 to 9.
